# EUROPEAN PATENT SPECIFICATION

(11) **EP 1 011 722 B1**
(45) Date of publication and mention of the grant of the patent: **09.11.2005**
(21) Application number: 98921925.8
(22) Date of filing: 13.05.1998
(51) Int. Cl.: A61K 39/39, A61K 9/14

(54) **SUBSTRATES MATERIALS WITH BOUND CYTOKINE-STIMULATING POLYSACCHARIDES OR BACTERIAL NUCLEIC ACIDS**
SUBSTRATMATERIALIEN MIT GEBUNDENEN CYTOKIN-STIMULIERENDEN POLYSACCHARIDEN ODER BAKTERIELLEN NUKLEINSÄUREN
MATERIAUX SERVANT DE SUBSTRATS ET COMPORTANT DES ACIDES NUCLEIQUES BACTERIENS OU DES POLYSACCHARIDES DE STIMULATION DE CYTOKINE LIES

(30) Priority: 16.05.1997 GB 9710010
(43) Date of publication of application: 28.06.2000
(73) Proprietor: FMC Biopolymer AS, 3013 Drammen (NO)
(72) Inventor: ESPEVIK, Terje, N-7036 Trondheim (NO); SKJAK-BRAEK, Gudmund, N-7016 Trondheim (NO)
(74) Representative: W.P. Thompson & Co.
(86) International application number: PCT/NO1998/000146
(87) International publication number: WO 1998/051342

(56) References cited:
- EP-A- 0 175 667
- EP-A- 0 506 325
- EP-A- 0 506 326
- WO-A-97/01330
- US-A- 4 460 575
- US-A- 4 650 675
- US-A- 4 701 521
- US-A- 5 171 738
- US-A- 5 457 187
- US-A- 5 504 079
- DIALOG INFORMATION SERVICES, File 155, MEDLINE, Dialog Accession No. 06775977, Medline Accession No. 91360008, ABE H., "Antitumor Effect of LPS Immobilized Beadsa"; & NIPPON GEKA GAKKAI ZASSHI, June 1991, 92 (6), p. 627-35.
- DIALOG INFORMATION SERVICES, File 34, SciSearch Dialog Accession No. 02410645, OTTERLEI M. et al., "Similar Mechanisms of Action of Defined Polysaccharides and Lipopolysaccharides - Characterization of Binding and Tumor-Necrosis-Factor-alpha Induction"; & INFECTION AND IMMUNITY, May 1993, Vol. 61, No. 5, p. 1917-1925.
- VACCINE, Volume 4, 1986, J. KREUTER et al., "Influence of the Particle Size on the Adjuvant Effect of Particulate Polymeric Adjuvants", pages 125-129.
- DIALOG INFORMATION SERVICES, File 34, SciSearch, BERNTZEN G. et al., "The Tumor Necrosis Factor-Inducing Potency of Lipopolysaccharide and Uronic Acid Polymers is Increased When They are Covalently Linked to Particles"; & CLINICAL AND DIAGNOSTIC LABORATORY IMMUNOLOGY, May 1998, Vol. 5, No. 3, p. 355-361.
- CHEMICAL ABSTRACT, Volume 125, No. 23, 2 December 1996, (Columbus, Ohio, USA), ALIKIN YU S. et al., "Prophylactictreatment of Viral Diseases in Fish Using Native RNA Linked to Soluble and Corpuscular Carriers", Abstract No. 292551; & J. FISH BIOL., 1996, 49 (2), 195-205.

## Description

This invention relates to polysaccharides and bacterial nucleic acids which are capable of stimulating an immune response, and more particularly to substrate materials which potentiate that immune response. In specific and preferred embodiments of the invention, low molecular weight fragments of longer chain immune- stimulating polysaccharides and bacterial nucleic acids which have only a low bioactivity, as compared to the parent substance from which they are derived, are potentiated sufficiently so as to permit their use in substitution for the parent compound.

It is known that different uronic acid polymers with a β1-4 glycosidic linkage are able to stimulate monocytes to produce tumour necrosis factor (TNF) through a membrane CD14 dependent manner (Espevik et al, Eur. J. Immunol. 23:255). Mannuronan (poly M) is the most potent of the β1-4 linked uronic acid polymers in inducing cytokine production. However, the cytokine stimulatory activity of mannuronan is dependent of the molecular weight of the polymer, and optimal cytokine induction is obtained when the MW is 50,000 or higher (Otterlie et al, Infect. Immun. 61: 1993m oages 1917-1925. However, there is a sharp decline in activity at lower molecular weights and all useful activity is lost at a molecular weight below 10,000 g/mol. Although there are no apparent toxic effects when high molecular weight mannuronan is injected into mice, nonetheless it is important to use a polymer size as small as possible for therapeutic purposes in order to promote more complete and rapid excretion of the injected material from the body. This requirement therefore conflicts with the desire to optimise the TNF stimulating activity.

It is also known that lipopolysaccharide (LPS) has a TNF-inducing ability which depends on the three- dimensional supramolecular structure (Rietschel et al, "Bacterial endotoxins: properties and structure of biologically active domains", Werlag Chemie, 1988, pl). These supramolecular structures depend on the amount and distribution of the acyl chains in the lipid A region of LPS, and when lipid A occurs in a cubic or inverted hexagonal structure an increased cytokine induction is observed, whereas a lamella structure gives no cytokine induction.

LPS as such is highly toxic but it can be delipidized by alkaline hydrolysis to form a detoxified LPS (D-LPS) from which the lipid A region, which is the main cause of the toxicity, has been removed. However, D-LPS has only a low ability to stimulate monocytes to produce TNF, despite retaining an intact polysaccharide portion.

Other members of the family of polymers of uronic acid are also known to stimulate monocytes to produce TNF or other cytokines. For instance, D-glucuronic acid (D-GlcA) has this property, although with less potency compared with mannuronan.

Polysaccharides from gram-negative and gram- positive bacteria such as lipoarabinomannan, lipoteichoic acid and peptidoglycans are also known to induce cytokines.

Another well known immune-stimulating polysaccharide is chitosan.

It is, however, to be understood that not all polysaccharides have the ability to stimulate monocytes.

Certain bacterial nucleic acids form another category of substances with the capability of stimulating an immune response.

We have now found, in accordance with the present invention, that the cytokine-inducing activity of many immune-stimulating polysaccharides and bacterial nucleic acids, as well as of lower molecular weight fragments thereof, can be improved by binding the polysaccharides to the surfaces of a substrate. This surprising discovery not only helps to overcome the problems discussed above with using mannuronan and D-LPS, for example, in therapy, but more generally opens up new therapeutic possibilities not hitherto available.

It is disclosed by Seljelid et al of the Institute of Medical Biology, University of Tromso, Norway in Scand. J. Immunol. 25, 55-60, (1987) that plastic microbeads derivatized with β-1,3-D glucan protect mice against pneumococcal and E. coli infections. These and other workers at the Institute of Medical Biology have subsequently shown that this protective effect is caused at least partially in part by stimulation of cytokine release (see, for example, Rasmussen et al, Journal of Cellular Biochemistry (1991) 46:60-68).

Nippon Geka Gakkai Zasshi 92 (1991) 627 discloses lipopolysaccharide (LPS) immobilized to polystyrene beads and its use for antitumor purposes. It was considered that LPS immobilized beads induced killer cells through cytokines.

US-A-5171 738 discloses LPS from the cell walls of gram-negative bacteria immobilized on a solid insoluble carrier and its use in treating tumours.

WO-A-97/01330 discloses polysaccharide-phospholipid conjugates and their use as adjuvants suitably formulated with antigen to provide vaccines.

EP-A-0 175667 discloses a macrophage stimulatory composition containing a water-insoluble β-1,3-bound glucan and a water-soluble carrier to which the glucan has been immobilized.

EP-A-0 506 326 discloses the use of β-1,4-polyuronates and their use for cytokine stimulation.

US-A-4460575 discloses a vaccinal complex comprising bacterial ribosomal RNA or fragments of bacterial ribosomal RNA on which are coupled from 1 to 5% by weight of a specific antigen of bacterial type

According to the present invention there is provided a pharmaceutically acceptable substrate material to a surface of which is chemically bound an cytokine-stimulating bioactive substance selected from the group consisting of polysaccharides, containing from 2 to 100 sugar units other than β-1,3-D glucan, and bacterial nucleic acids containing from 2 to 100 sugar units. The present invention also provides a method for potentiating the cytokine-stimulating effect of an immune-stimulating polysaccharide, other than β-1,3D glucan, or of an immune-stimulating bacterial nucleic acid, wherein said bioactive substance is contacted with a substrate so as to become bound to a surface thereof.

The present invention provides a new approach to potentiating the cytokine stimulating activity of polysaccharides and bacterial nucleic acids. In particular, to the best of our knowledge it has not hitherto been disclosed that lower molecular weight fragments of immune-stimulating polysaccharides and bacterial nucleic acids which have only relatively poor cytokine stimulating properties, as compared to their parent compound, may, by means of being bound to the surface of a substrate as disclosed herein, have their activity increased to a level which permits the use of the fragments in place of the parent compounds. The magnitude of the potentiation of the cytokine-stimulating activity which is achievable in accordance with this invention is illustrated in the Examples below.

Preferably the polysaccharides and nucleic acids contain from 10-30 sugar units. Polysaccharides which contain from 2 up to about 7 sugar units are often considered to be, and are termed, oligomers (oligo-saccharides).

Although, as already stated, the invention has particular applicability to the potentiation of polysaccharide and nucleic acid fragments, it may also be used to potentiate the cytokine-stimulating activity of the immune-stimulating polysaccharides and bacterial nucleic acids themselves.

It appears from our studies that the form and nature of the substrate which is used as carrier for the active polysaccharide or nucleic acid material is not especially critical. For some purposes, it is desirable that the substrate should be in particulate form, for example for intravenous or subcutaneous injection, but in other cases the substrate could take the form of a body for implantation in vivo. In yet other cases, the substrate could be a material over which, for instance, a fluid could be brought into contact to achieve a reaction with the bound bioactive material.

Similarly, a wide range of different natural or synthetic materials may be used as the substrate. It will, of course, be understood that the substrate material must not cause unwanted reactions in the environment in which it is to be used.

In the experiments which are described in the Examples below we used magnetic monodisperse polystyrene particles made by the active swelling technique described by Ugelstad et al in Progress in Polymer Science, 17, No. 1, 87-161 (1992). Epoxy groups were introduced on the surface of the particles, and different amounts of amino linkers were then coupled to the epoxy surface by the method of Hermanson et al in Immobilized Affinity Ligand Techniques, Academic Press, New York 1992.

For some purposes, it is preferred that the substrate should be constituted by materials capable of being absorbed in vivo. A wide range ofbioerodible and resorbable polymeric and other solid materials are known to the art.

Other natural or synthetic materials which could be used as the substrate in this invention will readily suggest themselves to those skilled in the art.

The size of substrate particles can vary widely, depending on the intended end use. For example, particles for subcutaneous injection can have a size up to 50 µm but preferably less than 20 µm, although for intravenous injection the particle size should be less than 5 µm The shape of the particles used as substrate material is not critical.

The bioactive polysaccharide or nucleic acid component may be linked to the substrate in many different ways. For instance, a polysaccharide may be linked covalently by coupling through hydroxyl groups (always present in carbohydrates), carbonyl groups, amine groups (amino sugars) and carboxyl groups (uronic acids), or through substituents such as sulphate or phosphate groups, as appropriate. If required, linkers for covalent coupling can be applied to a substrate surface lacking groups capable of entering into covalent bonding with the bioactive component.

Some examples of covalent coupling chemistry useful herein are:

### Carbonyl reactive chemistry

Carbonyl groups, either aldehydes or ketone on reducing sugars.

Aldehydes groups can also be introduced easily by periodate oxidation.

### Example: Hydrazide activation

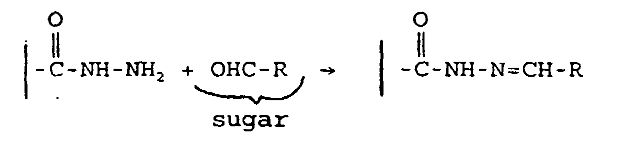

### Example: Reductive amination using cyanoborohydride

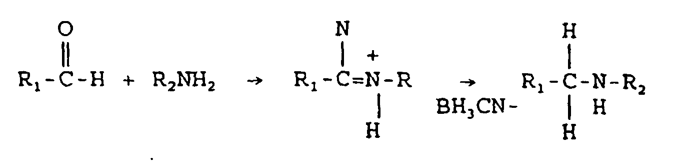

### Hydroxyl reactive chemistry

Example: Polysaccharides can be activated with CNBr which then easily can be coupled to any ligand containing primary amines.

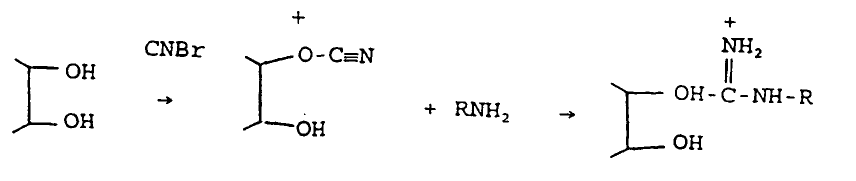

### Example: Epoxy activated surfaces react with hydroxyl or amine groups at high pH and are well suited for carbohydrate coupling.

### Carboxyl reactive chemistry

An example based on carbodiimide is described in the Examples below.

Although covalent coupling of the polysaccharide or nucleic acid is often preferred, it is also possible to bind the bioactive component to the substrate non- covalently. For example, coupling can be achieved by ionic or hydrophobic interactions, or by means of biospecificity (e.g. enzyme substrate antigen antibody).

The density of the bioactive polysaccharide or nucleic acid material bound on the surface of the particles or other substrate material is selected having regard to the intended use of the resulting composition. Generally, the density will lie in the range of 0. 1 to 100 ng/µm², preferably in the range 0.1 to 50 ng/µm².

There is some evidence that there may, for any given polysaccharide or nucleic acid, be an optimum density to achieve maximum cytokine production, but further work is needed to confirm whether or not this is the case.

Although we do not wish to be bound by theory, our work indicates that when M-blocks or detoxified LPS, for example, are bound to a substrate surface, multiple membrane receptors may be aggregated which can synergize the induction of TNF.

The substrate materials of this invention are expected to find many uses in medicine. For example short blocks of mannuronan covalently linked to biodegradable particles can be used as an immunostimulator for the treatment of various types of diseases, for instance those in which patients will benefit from an enhanced cellular immunity, achieved through increased cytokine production.

Further, substrate materials of this invention can be used as an immunostimulator for protecting a patient who is about to undergo major surgery from infection from gram-positive or gram-negative bacteria. Another potential use of the present materials is as an immunostimulator to protect a patient who is about to undergo radiation therapy which damages bone marrow cells. The administration of, for instance, poly M-bound particles in accordance with this invention will lead to enhanced hematopoiesis.

The substrate materials of this invention may also be used as targeting anti-cancer drugs. Tumour cells are known to produce inhibiting factors for macrophages, but this may be reversed or depressed through the administration of, for instance, poly M-bound particles which have a stimulating effect on macrophages.

Particles to which are bound polysaccharides and bacterial nucleic acids in accordance with this invention may be administered, for example, by intravenous or subcutaneous injection. For this purpose the particles can be formulated with a pharmaceutically acceptable injectable carrier, for example a physiological buffer.

The invention is illustrated by the Examples which follow.

### Materials and Methods

Poly M was isolated from agar colonies of *Pseudomonas aeruginosa* 8830, which was grown at 18°C as described by Gross et al, J. Phytophatol, 1983, 118:276: ¹⁴C-labelled fructose (Amersham, Buckinghamshire, England) was supplemented to the medium to make the alginate radioactive. The material was purified by a repeated combination of alkali treatment with 0.2 M NaOH at 45°C, precipitation with ethanol, and extraction of the precipitate by ethanol and chloroform. The polymer was dissolved in pyrogen-free water, filtered through 0.22 µm membrane filter (Millipore) and lyophilized. LPS contamination in poly M was measured by LAL assay (Chromogenix AB, Mölndal, Sweden). The level of endotoxin in the polymer was <0.25 ng/mg. The content of mannuronic acid was estimated to be 92% by ¹H-NMR spectroscopy Grasdalen et al, Carbohydr. Res. 1979, 68:23 and Grasdalen, Carbohydr. Res. 1979, 68:23), and the average molecular weight was estimated to be 350,000 g/mol by viscometry (Scott-Geräte). M-blocks (94% D-ManA) were prepared by hydrolysis of poly M for 1 hr at 100°C at pH 5.6 and 1 hour at 100°C at pH 3.8. This procedure yielded M-blocks with an average MW 5500 and 94% D-ManA. For some experiments M-blocks with an average MW of 3000 were produced by additional hydrolysis.

G-blocks (94% L-GulA and degree of polymerisation, 27) were isolated from colonies of *Azotobacter vinelandii* grown at 37°C with ¹⁴C-labelled fructose (Skjak-Bræk et al, Carbohydr. Res. 1982, 103:133). Such G-blocks do not have any immune- stimulating properties.

C60XY (β1-4 linked glucuronic acid [D-GlcA] was prepared by oxidation of cellulose at position C-6 (Painter, Corb. Res. 55, 95-103, 1977). The average MW was estimated from intrinsic viscosity measurement to be 30,000, and the degree of oxidation (88% D-GlcA and 12% D-Glc) was determined by titration (Nevell, Methods Carbohydr. Chem. 1963, 3:161 and Yackel et al, JACS 1942, 64:121).

Endotoxin contamination in the different polysaccharides was measured by the *Limulus* amebocyte lysate (LAL) assay (Chromogenix AB, Mölndal, Sweden). The estimated levels of endotoxin were as follows: M-blocks: 0.24 ng/mg; poly M: 0.25 ng/mg; G-blocks: 12.4 ng/mg; C60XY: 1.12 ng/mg.

Lipopolysaccharide and detoxified LPS (D-LPS) from smooth *Salmonella minnesola* were purchased from Sigma. D-LPS had been prepared by alkaline deacylation of LPS through the removal of the ester linked fatty acids (Ding et al, J. Med. Microbiol 1990, 31:95)

The characteristics of the polysaccharides are summarized in Table 1 below.

The characteristics of the polysaccharides are summarized in Table 1 below.

**Table 1.**

| Characteristics of the polyuronic acids used in this study. | | | |
|---|---|---|---|
| Polysaccharide | Source | Molecular weight | Monomer Composition |
| poly M | *P*. *a*.* | 350,000 | 92% D-ManA, 8% L-Gula |
| M-blocks | *P*. *a*.* | < 5,500 | 94% D-ManA, 6% L-GulA |
| G-blocks | *A*. *v*.** | 5,500 | 94% L-GulA, 6% D-ManA |
| C60XY | Cellulose | 30,000 | 88% D-GlcA, 12% D-Glc |

| | | | |
|---|---|---|---|
| *) *P*. a. = *Pseudomona aeruginosa* | | | |
| **) *A*. *v*. *= Actobacter vinelandii* | | | |

### Covalent coupline ofuronic acids and D-LPS to particles

Magnetic monodisperse polystyrene (PS) particles with epoxy groups (Ugelstad et al, Progress in Polymer Science, 1992, 17:87) were aminated as described by Hermanson et al (Immunobilized Affinity Ligand Techniques, Academic Press 1992). In some experiments hydrophilic bovine serum albumin (BSA, Sigma) particles were prepared according to the method described by Longo et al (J. Pharm. Sci. 1992, 71:1323). Uronic acids and D-LPS were coupled to magnetic monodisperse- or BSA particles through formation of amide bonds between the carboxylic groups on the uronic acids and primary amine groups on the particles. The coupling was carried out in 0.1 M phosphate buffer, pH 7.3, by adding carbodiimide EDC (1-ethyl-3-(3-dimethlaminpropyl)carbodiimide) and sulfo-NHS (N-hydroxysulfosuccinimide) as described by Staros et al (Anal. Biochem. 1986, 156:200). After linking the polysaccharide to the particles, they were extensively washed in 0.1 M phosphate buffer, pH 10 in order to remove noncovalently bound polysaccharide. For some experiments particles made of crosslinked bovine serum albmin were made. The amounts of M- and G-blocks covalently linked to the particles were estimated by measuring the radioactivity in a β-counter (Packard). The characteristics of the particles used and the amount of M-blocks and G-blocks coupled to them are given in Table 2 below.

**Table 2.**

| Characteristics of the beads used in this study and the amount of covalently linked M-blocks and G-blocks | | | | | |
|---|---|---|---|---|---|
| Particle type | φ [µm] | Surface [µm²] | Primary aminogroups on the surface [mmol/g] | Amount of M-blocks [ng/10⁶ beads] | Amount of G-blocks [ng/10⁵ beads] |
| PS 1 | 4.5 | N.D. | 0.65 | 33 | N.D. |
| PS 2 | 4.2 | 2.3 | 0.11 | 12 | 17 |
| PS 3 | 4.5 | 3.8 | 0.50 | 50 | 47 |
| PS 4 | 4.5 | 3.8 | 0.36 | 43 | 54 |
| BSA | 5-10 | N.D. | N.D. | 117 | N.D. |
| ND = not determined | | | | | |

### Monocyte cultivation

Monocytes were isolated from A⁺ blood buffycoat (The Blood Bank, University Hospital, Trondheim, Norway) as described by Bøyum (Scand. J. Immunol. 1976, 5:9). Monolayers of monocytes in 24-well culture plates (Costar, Cambridge, MA) were cultured in AIM serum-free medium (Gibco) with 1% glutamine and 40 µm/ml Garamycin. Different concentrations of particles and polysaccharides in solution were added to monocytes, and supernatants were harvested 8 hours later and assayed from TNF activity in the WHI clone 13 bioassay (Espevik et al, J. Immunol. Methods, 1986, 95:99).

### SW480/β-gal cultivation

Human colon adenocarcinoma cells, SW480/β-gal (donated by Dr. Gerald Ranges, Miles Inc., West Haven, CT, USA), contain a beta galactosidase (β-gal) gene under control of the cytomegalovirus (CMV) immediate early promoter/enhancer region (Galloway et al, Eur. J. Immunol 1992; 22:305). SW480/β-gal were grown in RPMI 1640 (Gibco Laboratories, Paisley, Scotland), supplemented with 2 mM L-glutamine, 10% heat-inactivated FCS (HyClone, Logan, UT, USA) and 40 µg/ml Garamycin (FCS medium). Stimulation with particulate and soluble forms of M-blocks and different forms of LPS was carried out in RPMI 1640 medium supplemented with glutamine, 20% human A⁺ serum (The Blood Bank, University Hospital ofTrondheim, Trondheim, Norway) and Garamycin (A⁺ medium). The β-galactosidase assay was performed essentially as described previously (Loegreid et al J. Biol. Chem. 1995. 270:25418). Substrate conversion was measured as optical density (OD) at 570 nm.

### TNF assay

TNF activity was determined by measuring its cytotoxic effect on the fibrosarcoma cell line WEHI 164 clone 13 as described by Espevik et al (J. Immunol. Methods 1986, 95:99). Dilutions of recombinant human TNF (donated by Dr. Refaat Shalaby, Genentech, South San Francisco, CA) were included as a standard. The TNF specificity of the assay was verified by use of a neutralizing Mab against recombinant human TNF (Liabakk et al, J. Immunol Methods, 1990, 134:253). The results are presented as pg/ml ± SD for triplicate determinations.

### Example 1

The purpose of this experiment was to test whether the TNF-inducing potency of poly M was affected by its form.

Radiolabelled poly M with MW of 350,000 was degraded by acid hydrolysis by the process described above to obtain polysaccharide fragments (M-blocks) with an MW of 5.5 kD. The resultant M-blocks were covalently linked by the procedure described above, to the two types of polystyrene particles, namely PS 2 and PS 3 (see Table 2 above).

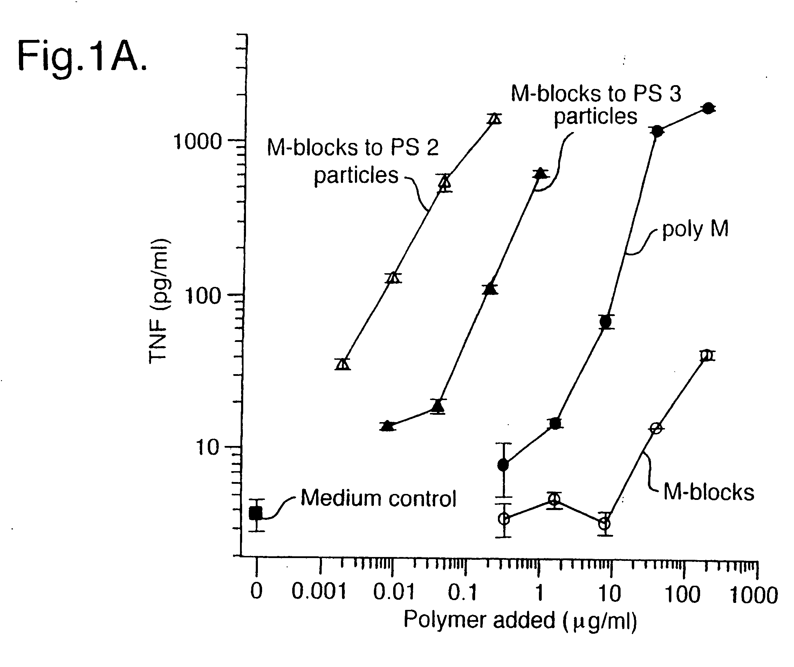

The TNF-inducing potency of the two particle- bound M-blocks was then determined by the TNF assay described above, and compared to that of unlinked M-blocks, and also to unhydrolysed poly M in phosphate- buffered saline solution. The results are shown in Fig. 1A.

As can be seen from Fig. 1A, reduction of the polymer size to 5.5 kD reduced the TNF inducing potency by a factor of 10-100. However, covalently linking the 5.5 kD M-blocks to PS 2 or PS 3 particles resulted in a 2500 and 60,000 times increase, respectively, in the TNF inducing potency compared to soluble M-blocks. Linking M-blocks to particles also potentiated the TNF response compared to poly M in solution.

The experiment was repeated, but with the amino groups on the particles substituted with carboxyl groups. It was found that this substitution did not change the TNF release from monocytes. This therefore indicates that the stimulatory effect of M-blocks linked to particles is not caused by a net negative charge on the particles or a non-specific reaction due to the coupling procedure.

### Comparative Example 1

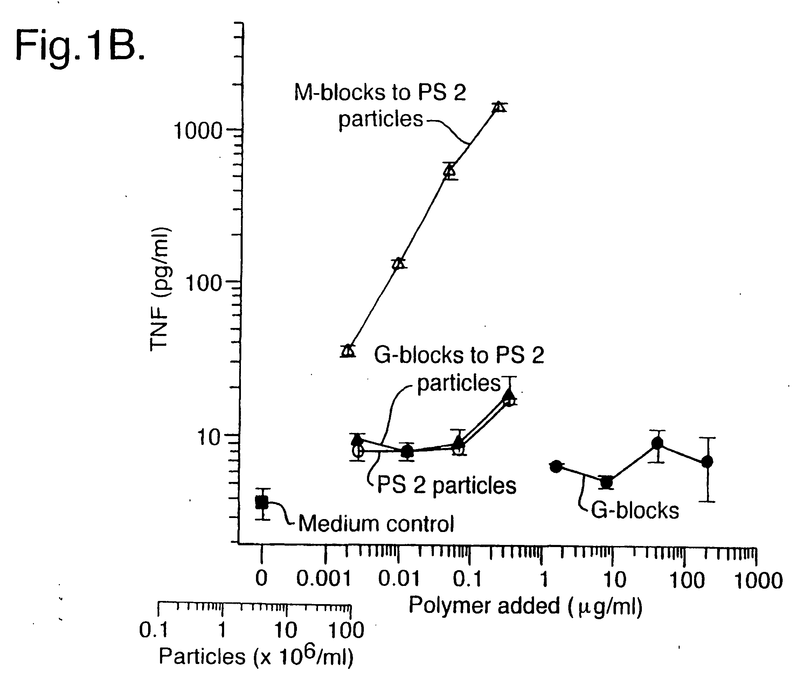

Example 1 was repeated but with 5.5 kD G-blocks prepared as described above. The results are shown in Fig. 1B.

It is seen by Fig. 1B that G-blocks in solution or linked to PS 2 particles did not induce the monocytes to produce TNF.

This experiment demonstrates that binding to a substrate a polysaccharide which inherently does not exhibit a capability of stimulating an immune response has no effect on this characteristic of the substance.

### Example 2

The TNF assay was repeated on LPS and detoxified LPS (both obtained from Sigma). The reagents were added as solutions. As in Example 1, M-blocks were used as a control.

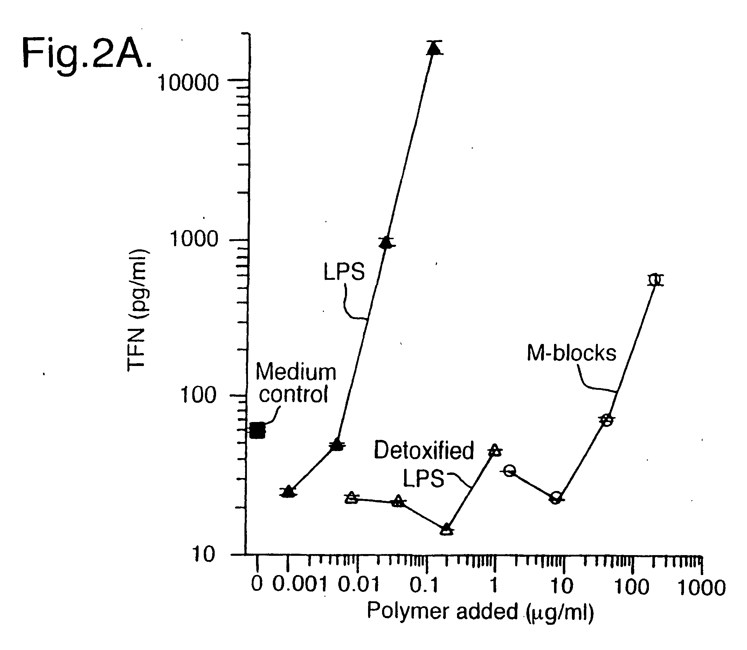

As shown in Fig. 2A, when tested on monocytes in solution under serum free condition it was found that the detoxified LPS (D-LPS) up to 1 µg/ml did not induce monocytes to produce TNF, whereas the untreated LPS gave a strong TNF response.

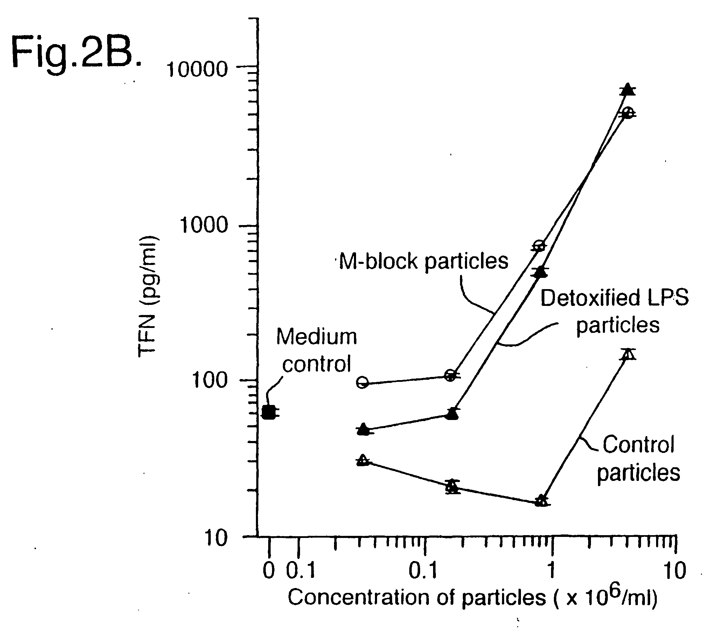

However, when in accordance with the present invention the D-LPS was linked covalently to PS 1 particles (see Table 2 above) and the TNF assay was repeated it was found that there was a high production of TNF, comparable with the M-block particles of Example I (Fig. 2B).

Since the molecular weights of M-biocks and D-LPS are comparable, and since D-LPS also was linked to the particles by amine bonds implicate that the amount of D-LPS bound is equal or less than the amount of M-blocks bound to the particles. The data thus shows that lower MW fragments from polysaccharides are very potent TNF inducers when presented for monocytes on the surface of particles.

### Comparative Example 2

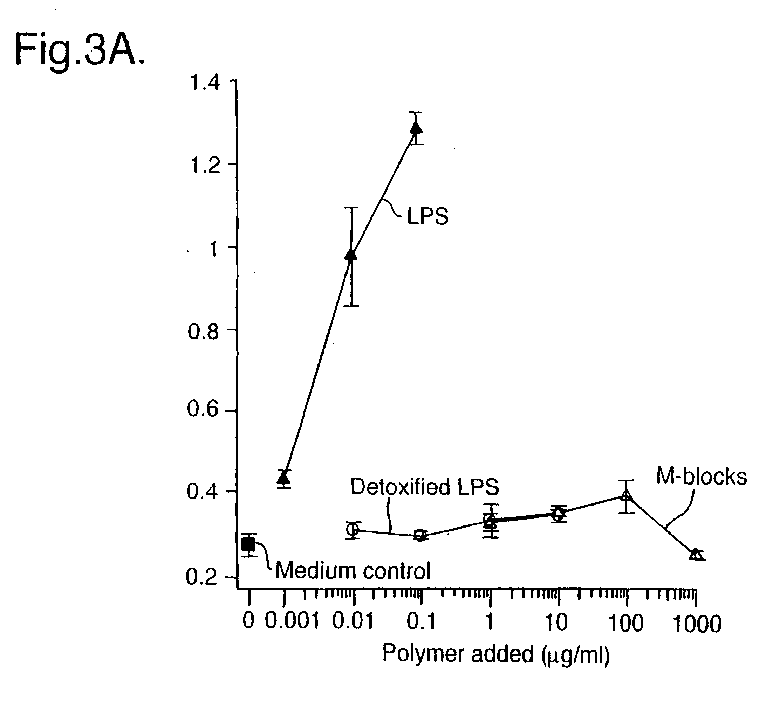

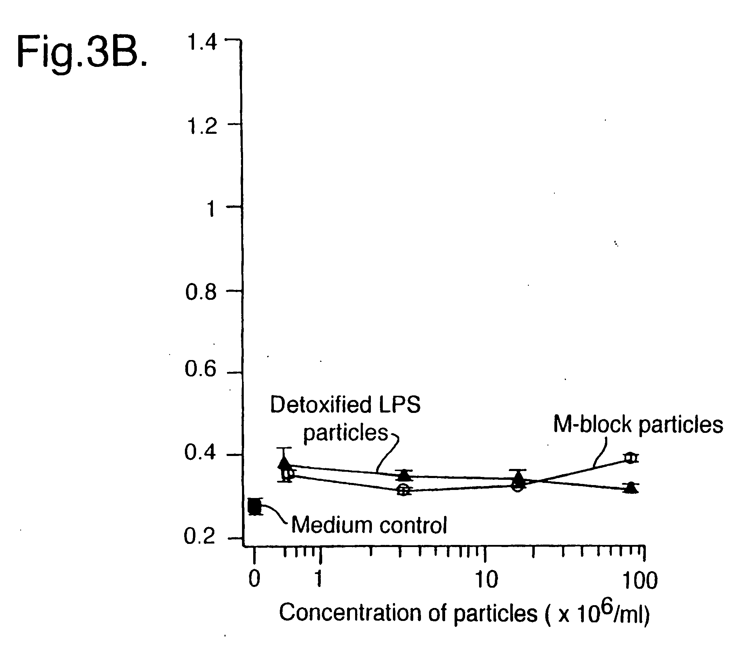

The SW480-βgal cells do not express functional membrane CD14, but respond to LPS in the presence of serum. It was therefore of interest to study if D-LPS or M-blocks, either in solution or linked to particles according to the present invention, were able to activate these cells. As can be seen from Fig. 3A, the complete LPS gave a strong and dose related activation of the human CMV promoter in the SW480-βgal cells, whereas D-LPS or M-blocks in solution had no stimulatory effect. In addition, M-block and D-LPS bound to PS 1 polystyrene particles had no stimulatory effect on this cell type (Fig. 3B). This data indicate that M-block and D-LPS particles have a preference in stimulating membrane CD14 positive monocytes and no LPS responsive cells which lack membrane CD 14.

### Example 3

The TNF assay was repeated with another member of the uronic acid family, D-glucuronic acid (D-GlcA) polymer. Such polymers are known to stimulate monocytes to produce TNF in a CD14-dependent manner, although with less potency as compared to poly M.

A polymer consisting of 88% D-GlcA and 12% D-Glc, and with an MW of 30,000, was prepared by oxidizing cellulose by the method described above.

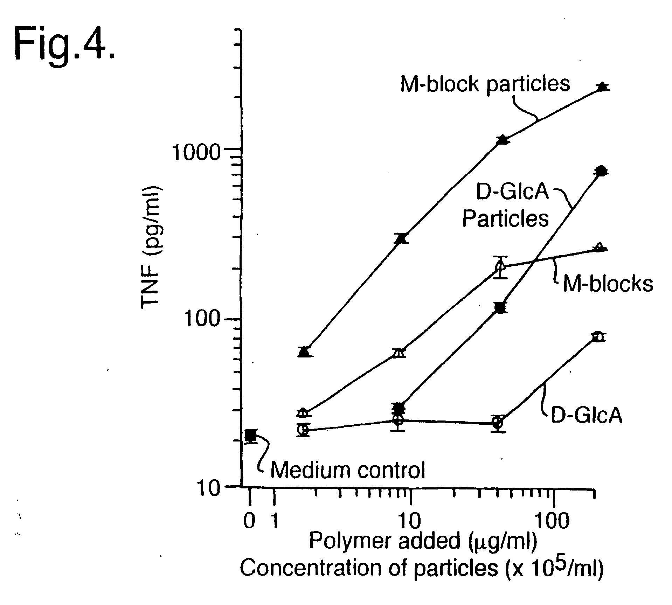

The TNF assay was conducted both on the D-GlcA polymer in phosphate buffered saline solution (1-2 ml/mg) and also on the polymer covalently bound to PS 2 particles in accordance with the present invention. The results are shown graphically in Fig. 4.

It will be noted that the D-GlcA polymer in solution resulted in a low production of TNF However, the D-GlcA bound to the PS 2 polystyrene particles showed a marked increase in TNF production.

In Fig. 4, the results for the M-blocks are shown for comparison purposes.

### Example 4

The purpose of this experiment was to test whether TNF-stimulating activity would be exhibited by mannuronan fragments of very low molecular weight if they were covalently bound to bioabsorbable particles in accordance with this invention.

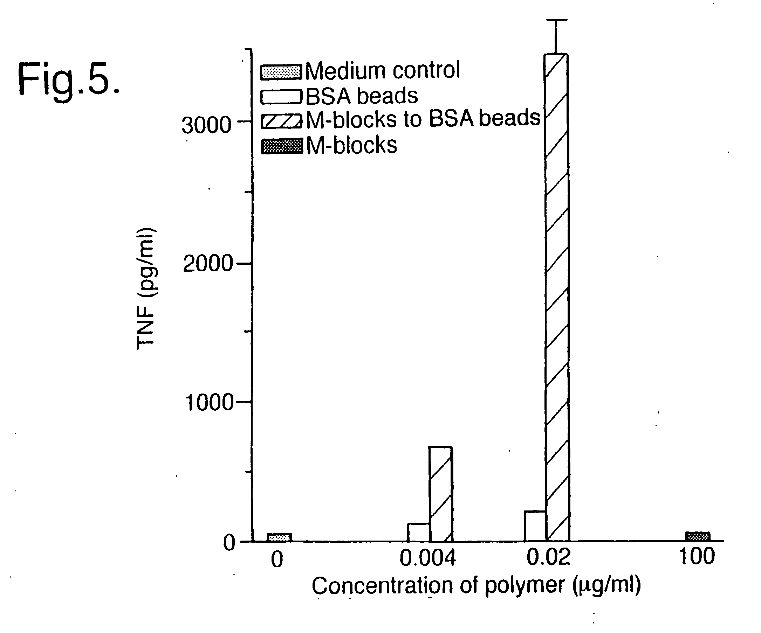

The TNF assay was therefore repeated on M-blocks with an MW of around 3,000, prepared as described hereinabove. The resulting low molecular weight oligomer was covalently bound to BSA particles (see Table 2 above) by the method described above. The results of the TNF assay on the particle-bound oligomer are shown in Fig. 5.

It will be seen from Fig. 5 that adding soluble M-blocks to monocytes did not result in production of TNF even at a concentration of 100 µg/ml. However, adding M-block-BSA particles in accordance with this invention resulted in more than 1 ng/ml of TNF at a polymer concentration equivalent to 0.02 µg/ml. Even at a polymer concentration equivalent to 0.004 µg/ml there was significant production of TNF.

## Claims

1. A pharmaceutically acceptable substrate material to a surface of which is chemically bound an cytokine-stimulating bioactive substance selected from the group consisting of polysaccharides containing from 2 to 100 sugar units, other than β-1,3-D glucan, and bacterial nucleic acids containing from 2 to 100 sugar units.

2. A substrate material according to claim 1, in which said bioactive substance is a polysaccharide containing from 2-100 sugar units.

3. A substrate material according to claim 2, wherein said polysaccharide contains from 10-30 sugar units.

4. A substrate material according to any one of the preceding claims, wherein said bioactive substance is a 1-4 linked uronic acid polymer.

5. A substance material according to claim 4, wherein said bioactive substance consists of more than 80% mannurionic acid residues.

6. A substrate material according to any one of claims 1 to 3, wherein said bioactive substance is detoxified lipopolysaccharide.

7. A substrate material according to any one of the preceding claims, wherein said substrate is in the form of particles.

8. A substrate material according to claim 7, wherein said particles have a size up to 50 µm.

9. a substrate material according to claim 8, wherein said particles have a size up to 5 µm.

10. A substrate material according to any one of claims 7 to 9, wherein said particles are surface-modified polystyrene particles or albumin particles.

11. A substrate material according to any one of the preceding claims, wherein said bioactive substance is covalently coupled to said substrate surface.

12. A pharmaceutical composition suitable for injection, comprising a substrate material according to any one of claims 7 to 10 and a pharmaceutically acceptable injectable carrier therefor.

13. A method for potentiating the cytokine-stimulating effect of an immune-stimulating bioactive substance selected from the group consisting of polysaccharides, containing from 2 to 100 sugar units other than β-1,3-D glucan, and bacterial nucleic acids containing from 2 to 100 sugar units, wherein said bioactive substance is contacted with a pharmaceutically acceptable substrate so as to become chemically bound to a surface thereof.

14. A method according to claim 13, wherein said bioactive substance is as defined in any one of claims 2 to 6.

15. A method according to claim 13 or claim 14, wherein said substrate is as defined in any one of claims 7 to 10.

16. A method according to any one of claims 13 to 15, wherein said bioactive substance is covalently linked to said substrate surface.

## Patentansprüche

1. Pharmazeutisch verträgliches Substratmaterial an dessen Oberfläche eine Cytokin-stimulierende bioaktive Substanz chemisch gebunden ist, die aus der Gruppe ausgewählt ist, bestehend aus Polysacchariden, die von 2 bis 100 Zuckereinheiten mit Ausnahme von β-1,3-D-Glucan enthalten, und bakterielle Nukleinsäuren, die von 2 bis 100 Zuckereinheiten enthalten.

2. Substratmaterial nach Anspruch 1, worin die genannte bioaktive Substanz ein Polysaccharid darstellt, das von 2 bis 100 Zuckereinheiten enthält.

3. Substratmaterial nach Anspruch 2, worin das genannte Polysaccharid von 10 bis 30 Zuckereinheiten enthält.

4. Substratmaterial nach einem der vorangehenden Ansprüche, worin die genannte bioaktive Substanz ein 1 - 4 verknüpftes Uronsäure-Polymer darstellt.

5. Substanzmaterial nach Anspruch 4, worin die genannte bioaktive Substanz aus mehr als 80 % Mannuronsäureresten besteht.

6. Substratmaterial nach einem der Ansprüche 1 bis 3, worin die genannte bioaktive Substanz detoxifiziertes Lipopolysaccharid darstellt.

7. Substratmaterial nach einem der vorangehenden Ansprüche, worin das genannte Substrat in der Form von Partikeln vorliegt.

8. Substratmaterial nach Anspruch 7, worin die genannten Partikel eine Größe bis zu 50 µm aufweisen.

9. Substratmaterial nach Anspruch 8, worin die genannten Partikel eine Größe bis zu 5 µm aufweisen.

10. Substratmaterial nach einem der Ansprüche 7 bis 9, worin die genannten Partikel Oberflächen-modifizierte Polystyren-Partikel oder Albumin-Partikel darstellen.

11. Substratmaterial nach einem der vorangehenden Ansprüche, worin die genannte bioaktive Substanz kovalent an die genannte Substratoberfläche gekoppelt ist.

12. Pharmazeutische Zusammensetzung, die zur Injektion geeignet ist, umfassend eine Substratmaterial nach einem der Ansprüche 7 bis 10 und einen pharmazeutisch verträglichen injizierbaren Träger dafür.

13. Verfahren zum Potenzieren der Cytokin-stimulierenden Wirkung einer immunstimulierenden bioaktiven Substanz, die aus der Gruppe ausgewählt ist, bestehend aus Polysacchariden, die von 2 bis 100 Zuckereinheiten mit Ausnahme von β-1,3-D-Glucan enthalten, und bakterielle Nukleinsäuren, die von 2 bis 100 Zuckereinheiten enthalten, worin die genannte bioaktive Substanz mit einem pharmazeutisch verträglichen Substrat kontaktiert wird, um auf diese Weise an eine Oberfläche davon chemisch gebunden zu werden.

14. Verfahren nach Anspruch 13, worin die genannte bioaktive Substanz wie nach einem der Ansprüche 2 bis 6 definiert ist.

15. Verfahren nach Anspruch 13 oder 14, worin das genannte Substrat wie nach einem der Ansprüche 7 bis 10 definiert ist.

16. Verfahren nach einem der Ansprüche 13 bis 15, worin die genannte bioaktive Substanz mit der genannten Substratoberfläche kovalent verknüpft ist.

## Revendications

1. Matériau substrat pharmaceutiquement acceptable à une surface duquel est chimiquement liée une substance bioactive stimulant la cytokine choisie parmi le groupe constitué des polysaccharides contenant de 2 à 100 unités de sucre, autres que le β-1,3-D glucane, et des acides nucléiques bactériens contenant de 2 à 100 unités de sucre.

2. Matériau substrat selon la revendication 1, dans lequel ladite substance bioactive est un polysaccharide contenant de 2 à 100 unités de sucre.

3. Matériau substrat selon la revendication 2, dans lequel ledit polysaccharide contient de 10 à 30 unités de sucre.

4. Matériau substrat selon l'une quelconque des revendications précédentes, dans lequel ladite substance bioactive est un polymère d'acide uronique lié par des liaisons 1-4.

5. Matériau de substance selon la revendication 4, dans lequel ladite substance bioactive est composée de plus de 80 % de résidus d'acide mannuronique.

6. Matériau substrat selon l'une quelconque des revendications 1 à 3, dans lequel ladite substance bioactive est un lipopolysaccharide détoxifié.

7. Matériau substrat selon l'une quelconque des revendications précédentes, dans lequel ledit substrat est sous la forme de particules.

8. Matériau substrat selon la revendication 7, dans lequel lesdites particules ont une taille allant jusqu'à 50 µm.

9. Matériau substrat selon la revendication 8, dans lequel lesdites particules ont une taille allant jusqu'à 5 µm.

10. Matériau substrat selon l'une quelconque des revendications 7 à 9, dans lequel lesdites particules sont des particules de polystyrène modifiées en surface ou des particules d'albumine.

11. Matériau substrat selon l'une quelconque des revendications précédentes, dans lequel ladite substance bioactive est couplée par covalence à ladite surface du substrat.

12. Composition pharmaceutique adaptée pour une injection, comprenant un matériau substrat selon l'une quelconque des revendications 7 à 10 et un véhicule injectable pharmaceutiquement acceptable pour ce matériau.

13. Procédé pour potentialiser l'effet stimulant la cytokine d'une substance bioactive immunostimulante choisie parmi le groupe constitué des polysaccharides contenant de 2 à 100 unités de sucre autres que le β-1,3-D glucane, et des acides nucléiques bactériens contenant de 2 à 100 unités de sucre, dans lequel ladite substance bioactive est mise en contact avec un substrat pharmaceutiquement acceptable de façon à se retrouver chimiquement liée à une surface de celui-ci.

14. Procédé selon la revendication 13, dans lequel ladite substance bioactive est telle que définie dans l'une quelconque des revendications 2 à 6.

15. Procédé selon la revendication 13 ou 14, dans lequel ledit substrat est tel que défini dans l'une quelconque des revendications 7 à 10.

16. Procédé selon l'une quelconque des revendications 13 à 15, dans lequel ladite substance bioactive est liée par covalence à ladite surface du substrat.
